Europäisches Patentamt

**European Patent Office**

**Office européen des brevets**

(11) Publication number: **0 314 095 B1**

# EUROPEAN PATENT SPECIFICATION

(45) Date of publication of patent specification: **09.12.92**

(51) Int. Cl.⁵: **A61K 47/00**, A61K 35/16, A61K 47/02

(21) Application number: **88117833.9**

(22) Date of filing: **26.10.88**

(54) **Plasma and recombinant protein formulation in high ionic strength media.**

(30) Priority: **29.10.87 US 114314**

(43) Date of publication of application:
**03.05.89 Bulletin 89/18**

(45) Publication of the grant of the patent:
**09.12.92 Bulletin 92/50**

(84) Designated Contracting States:
**AT BE CH DE ES FR GB GR IT LI LU NL SE**

(56) References cited:
**EP-A- 0 106 269**
**EP-A- 0 187 712**
**EP-A- 0 237 981**

(73) Proprietor: **RHONE-POULENC RORER INTER-NATIONAL (HOLDINGS) INC.**
**40 Cape Henlopen Drive**
**Lewes, Delaware 19958(US)**

(72) Inventor: **Lee, Ted C.K.**
**9708 Ceralene Drive**
**Fairfax, VA(US)**
Inventor: **Hrinda, Michael E.**
**9718 Swift Creek Court**
**Fairfax Station, VA(US)**

(74) Representative: **Brauns, Hans-Adolf, Dr. rer. nat. et al**
**Hoffmann, Eitle & Partner, Patentanwälte Arabellastrasse 4**
**W-8000 München 81(DE)**

**Description**

This invention relates to stable factor VIII formulations. More particularly, high purity factor VIII protein is formulated in high ionic strength media for administration to patients suffering from haemophilia type A.

Antihaemophilic factor or factor VIII procoagulation activity protein (hereinafter factor VIII) functions to correct the clotting defect in hemophilia type A plasma. Accordingly, factor VIII preparations are extensively used for the purpose of supplying factor VIII to hemophilic patients.

An important concern associated with the use of factor VIII and other therapeutic agents derived from biological sources is the transmission of diseases, especially viral diseases. Prevalent viral contaminants include hepatitis B virus (HBV), non-A, non-B hepatitis virus (NANBV), and HTLV III/LAV/HIV which cause AIDS. In order to ensure that products produced from biological sources are virus-safe, various methodologies have been proposed for virus inactivation. However, most plasma protein preparations are unstable and require special care to prevent denaturation, alteration and loss of activity during the virus inactivation process. One approach to prevent denaturation and other alteration of plasma proteins utilizes additives during the pasteurization process. Representative examples follow.

USP No. 4,440,679 (Fernandes et al.) describes a method wherein therapeutically active proteins are pasteurized by mixing the protein composition with a pasteurization- stabilizing amount of a polyol prior to pasteurization.

USP No. 4,297,344 (Schwinn et al.) discloses a process for the stabilization against heat of the coagulation factors II, VIII, XIII, antithrombin III and plasminogen in aqueous solution, which comprises adding to the solution both an aminoacid and one or more of a monosaccharide, an oligosaccharide or a sugar alcohol.

USP No. 4,585,654 (Landaburu et al.) pertains to a process of inactivating viruses in plasma protein solutions by heating the same in the presence of a polyol, a surface active agent and a chelating agent.

USP No. 4,446,134 (Naito et al.) is drawn to a virus-inactivating process in which factor VIII is heated in an aqueous solution in the presence of one principal stabilizer of neutral amino acids, monosaccharides, oligosaccharides, and sugar alcohols and an auxiliary stabilizer of salts of hydrocarbon and hydroxyhydrocarbon carboxylic acids.

These processes aim at destroying the potential viral and bacterial infectivity of the preparations while substantially maintaining their desired biological activity. As much, they represent significant steps toward the provision of satisfactory plasma protein products to patients.

In order to be administrable, the plasma protein products need to be formulated with suitable compounds, lyophilized for storage and ready for reconstitution. Before formulating, the additives used during the pasteurization process are removed and their stabilizing/protecting effect is no longer present to prevent loss of activity. Applicants have encountered degradation problems with factor VIII both during lyophilization and upon reconstitution with normal saline solution. To eliminate the effects of residual stabilizing agents and/or other materials used in the prior art during the production or pasteurization, a highly purified factor VIII was used to study degradation occurring during lyophilization and reconstitution such as that produced by the teaching of USP No. 4,361,509. The method there disclosed provides for about one thousand-fold purification of factor VIII obtained from a commercial concentrate using an antibody column. The subsequent purification step by an Aminohexyl-Sepharose® column chromatography further increases purity by 2 to 3-fold resulting in factor VIII activity of 2,300 units per mg of protein.

Elution of factor VIII from the Aminohexyl-Sepharose® is accomplished by the use of calcium chloride solution having a concentration of from 0.25 to 0.5M. This solution, having such high concentration of calcium chloride is not suitable for injection to the patient. More importantly, upon lyophilization, a drastic loss of factor VIII was observed.

To remedy the problems, an isotonic solution was prepared by dialyzing factor VIII contained in said calcium chloride solution against 0.15M sodium chloride, 5 mM calcium chloride and 3mM histidine at pH 6.8. Upon testing, a drastic loss of factor VIII was again observed.

It has now been discovered that factor VIII as well as other plasma and recombinant proteins, can be formulated with physiologically acceptable compounds for stabilization against loss of activity during storage in liquid state, lyophilization, storage in the lyophilized state and reconstitution preceding administration to patients.

In accordance with the present invention plasma and recombinant protein formulations are provided which are stable, and upon reconstitution, are ready for administration into patients. The formulations comprise at least one particular protein as the active ingredient for therapeutic use and a high ionic strength media. The amount of protein present in a formulation is based on its known activity against the ailments to be treated and will vary from protein to protein, their concentration and state of purity. The high ionic strength media is an aqueous solution of and comprises:

(a) from 0.35M to 1.2M sodium chloride or potassium chloride or mixtures thereof and preferably 1M sodium chloride;

(b) from 1.5mM to 40 mM and preferably 3.5 to 15 mM calcium chloride; and

(c) from 1 mM to 50 mM and preferably 2 to 10 mM histidine as buffer ion.

The pH of the media should be from 6.0 to 7.6 and preferably 7.0.

Optionally, up to 10% w/v of sugars, such as mannitol, sucrose and maltose, may be added to the formulations of the present invention for lyophilization.

The formulation is lyophilized and is reconstituted to comprise:

(a) from 0.35 to 1.2M sodium chloride or potassium chloride or mixtures thereof and preferably 1M sodium chloride;

(b) from 1.5 mM to 40 mM and preferably 3.5 to 15 mM calcium chloride; and

(c) from 1 mM to 50 mM and preferably 2 to 10 mM histidine as buffer ion.

The present invention is also directed to a composition for stabilizing from 2 ml to 2000 ml of an aqueous solution of a lyophilized plasma protein, comprising:

from 0.04 to 141 g sodium chloride, from 0.05 g to 179 g potassium chloride, or mixtures thereof;

from 0.0003 g to 8.9 g calcium chloride; and

from 0.0003 g to 15.6 g histidine.

The formulations containing 2 to 500 units of factor VIII per ml of solution have been found effective for the treatment of hemophilia.

The present invention encompasses proteinaceous materials and products in the biomedical field intended for use in the human or animal body for biomedical or therapeutic purposes as well as non-therapeutic experimental purposes. Contemplated materials and products include but are not limited to:

Blood fractions such as antihemophilic factor (Smith, J.K and Bidwell, E. (1979) Clinics in Haemotol. 8, pp. 184-205);

Prothrombin complex, i.e., Factors II, VII, IX and X (Chandra, S. and Brummelhuis, H. G. J. (1981) Vox Sang. 41, pp. 259-273);

Protein C. (Steuflo, J. (1976) J. Biol. Chem. 251, pp. 355-363 and Bajaj, S. P. et al. (1983) Prep. biochem. 13 pp. 191-214); Protein S (diScipio, R.G., et al. (1977) Biochem. 16, pp. 698-706;

Antithrombin III (Rosenberg, R.D., and Damus, P.S. (1973) J. Biol. Chem. 248, pp. 6490-6505;

Gamma Globulin (Oncley et al. (1949) J. Amer. Chem. Soc. 71, pp. 541-550;

Biological materials and products derived by recombinant DNA techniques and produced in bacteria, fungi, or mammelian cell culture systems (Vane, J. and Cuatrecases, P. (1984), Nature 312, pp. 303-305 and Meniatis, T. et al. (1982), Molecular cloning: A Laboratory Manual, (Old Spring Harbor, NY).

These products and materials are available from various commercial sources or can be produced by using well-known preparative techniques. For example, blood fractions and blood proteins can be obtained from human blood plasma by fractionation according to known techniques such as, for example, the alcohol fractionation of Cohn described in USP No. 2,390,074 and the Journal of the American Chemical Society Vol, 68, p.459 (1946). These methods as well as other techniques are summarized in "The Plasma Proteins", second edition, Vol. III, pp. 548-550, Academic Press, New York, NY (1977).

While the invention is applicable to these and other similar products and materials, it will be described in detail in reference to factor VIII procoagulant activity protein produced according to USP No. 4,361,509. The method therein disclosed is capable of producing highly purified and concentrated factor VIII which is effective in the treatment of haemophelia, having more than two thousand units of factor VIII procoagulant activity per mg of protein. However, the product as obtained by the process is unstable during lyophilization and upon reconstitution. Furthermore, the high calcium ion solution containing the factor is undesirable for administration to the patients. The following examples and tests will further illustrate the invention.

Example 1

The rate of factor VIII degradation under isotonic conditions was studies. Factor VIII, obtained by the process of USP No. 4, 361,509, in buffered 500mM calcium chloride solution was dialyzed against 1M sodium chloride, 0.035 M calcium chloride and 3 mM histidine at pH 6.8, for salt exchange, and then was lyophilized. Reconstitution of the lyophilized material was made to 0.167 M sodium chloride, 5.8 mM calcium chloride, and 3 mM histidine by adding a 6-fold volume of 2.5 mM histidine, at pH 6.8, over the pre-lyophilization volume. The time dependent decay of factor VIII activity was determined by a two stage assay method which is essentially the same as the methods described by Newman, J., Johnson A. J., Karpatkin, S. and Puszkin, S. (1971), Br.J. Haemotol. 21, pp. 1-20. The results are shown in Table I.

TABLE I

| Time Dependent Decay of Factor VIII Activity under Isotonic Conditions | | |
|---|---|---|
| Time (Minutes) | Factor VIII Activity (Total Unit) | % Decay |
| 0 (At reconstitution) | 21 | 0 |
| 15 | 17 | 18 |
| 30 | 14 | 32 |
| 60 | 10 | 52 |

Example 2

A series of dialysis experiments were performed. Factor VIII in 500 mM $CaCl_2$ mM histidine, 0.1 M lysine hydrochloride, at pH 6.8, was dialyzed against histidine buffer solutions containing various concentrations of sodium or potassium chloride and calcium chloride. Samples were assayed by the two stage assay method referred to in Example 1. The particulars and results are shown in Tables II, III, IV and V.

TABLE II

| Effect of $CaCl_2$ Concentration on Factor VIII Activity in 0.15M NaCl, 0.1M Lysine HCl, pH 6.8 | | |
|---|---|---|
| mM ($CaCl_2$) | Factor VIII at Reconstitution U/M1 | Percent |
| 500 | 41.8 | 100 |
| 50 | 20.9 | 50 |
| 5 | 18.0 | 43 |

TABLE III

| Effect of $CaCl_2$ Concentration on Factor VIII Activity in 0.15M NaCl, 0.1M Lysine HCl, pH 6.8 | | |
|---|---|---|
| mM ($CaCl_2$) | Factor VIII 4 days after Dialysis U/M1 | Percent |
| 500 | 39.5 | 100 |
| 50 | 25.5 | 64 |
| 5 | 9.5 | 24 |

TABLE IV

| Effect of $CaCl_2$ Concentration on Factor VIII | | | |
|---|---|---|---|
| M (NaCl) | M ($CaCl_2$) | U/M1 | Percent |
| 0.15* | 0.5000* | 33.0 | 100 |
| 1.00 | 0.0005 | 18.3 | 55 |
| 0.15 | 0.0050 | 0 | 0 |
| 0.05 | 0.0050 | 0 | 0 |
| 0.005 | 0.0050 | 0 | 0 |

*The control contained 0.1M Lysine, the rest of the samples contained 3mM histidine, pH 6.8.

4

TABLE V

| Factor VIII Activity in Various Concentration of CaCl$_2$ and NaCl | | |
|---|---|---|
| (NaCl) mM | (CaCl$_2$) mM | Factor VIII Activity Percent |
| 1.00 | 5 | 100 |
| 0.70 | 5 | 91 |
| 0.15 | 22 | 75 |
| 0.15 | 10 | 75 |
| 0.15 | 15 | 58 |
| 0.45 | 5 | 32 |
| 0.15 | 5 | 0 |

The following example illustrates the result obtained by the present invention.

Example 3

Factor VIII (43 u/ml), prepared by the process of USP No. 4,361,509, was placed in IM sodium chloride, 5 mM calcium chloride, 3 mM histidine, at pH 7.0, and lyophilized. Reconstitution was made with 2.2 fold volume of water over the pre-lyophilization volume of factor VIII. The reconstituted solution contained: 0.45 M sodium chloride, 2.3 mM calcium chloride and 1.4 mM histidine. The recovery of factor VIII was measured by the two stage assay method referred to in Example 1. The results are shown in Table VI.

TABLE VI

| Time (Minutes) | Factor VIII Activity u/ml |
|---|---|
| 0 | 20.0 |
| 20 | 19.5 |
| 45 | 19.7 |
| 60 | 19.5 |
| 120 | 19.5 |
| 150 | 19.4 |
| 180 | 19.3 |
| 210 | 19.2 |

Example 4

Preparation of Solution of Cryoprecipitate

The starting material used for the preparation of factor VIII was cryoprecipitate of human plasma. Each kg of cryoprecipitate was placed in 1.4 kg of cold pyrogen-free (PF) water. Sixty ml each of 12% w/v glycine and 16% w/v sodium chloride was added to the mixture. The mixture was placed in a 37°C water bath to dissolve most of the cryoprecipitate and agitated to extract the factor VIII in to the solution. The pH of the mixture was titrated from 7.45 to 6.95 with 30 ml of 1 N acetic acid. Sixty-five g of Rehsorptar® (2% w/v aluminum hydroxide gel, Armour Pharmaceutical Company, Kankakee, Illinois) were added to the mixture and mixed for 20 minutes to absorb vitamin K-dependent clotting factors. The mixture was centrifuged at 3,000 xg for 20 minutes and the supernatant was collected. Fourteen ml of 1.5 M sodium citrate were added to the supernatant. The pH of the mixture was 7.40. The mixture was treated with Rehsorptar® once again and centrifuged as above. The resultant protein solution was stored at -40°C until used.

Isolation of Factor VIII by Monoclonal Anti-von Willebrand Factor Antibody Column Chromatography

The affinity column matrix was prepared by conjugation of monoclonal anti-von Willebrand Factor antibody to Sepharose® gel (4 g of antibody per 1 g of Sepharose® gel). Thawed cryoprecipitate solution, 4.8 1 (18,280 units of factor VIII) were applied to a 1.3 1 (9 x 21 cm) size of the antibody column, which was equilibrated with factor VIII buffer (o.15 M sodium chloride, 0.1 M lysine hydrochloride, 0.02 M histidine, pH 6.8). The flow rate of the loading was 15 ml per minute. The column was then washed with 3 column volumes of the factor VIII buffer. A total of 4,340 units was not bound to the column. The factor VIII was eluted from the antibody column with 0.5 M calcium chloride in the factor VIII buffer (flow rate: 21 ml/minute). The recovered factor VIII activity was 7,610 units in 0.68 1 of the column eluate.

Concentration, Formulation, Lyophilization and Reconstitution

The recovered factor VIII was concentrated in an Amicon® Hollow Fiber System (Type HIX 50-20, 2.5 cm x 20 cm long). The concentrated factor VIII, 50 g (56 u/ml, 2,800 units), was dialyzed at 4°C overnight against a formulation buffer which consisted of 1 M sodium chloride, 5 mM calcium chloride, 3 mM histidine, pH 7.0, with 2 changes. The dialyzed material, 46 u/ml, was lyophilized. The reconstitution of the lyophilized material was made to 0.45 M sodium chloride, 2.3 mM calcium chloride, 1.4 mM histidine, pH 7.0 by addition of 2.2 volumes of water for injection (WFI) over the pre-lyophilized volume. The reconstituted material was remarkably stable over a period of 3 hours and the recovery of the factor VIII activity was about 91%. The assay values (18 to 20 u/ml) were essentially the same, within experimental error, as shown in Table VII.

TABLE VII

| Time (Minutes) After Reconstitution | 9 | 21 | 33 | 46 | 68 | 95 | 125 | 191 |
|---|---|---|---|---|---|---|---|---|
| Activity (u/ml) | 18 | 19 | 20 | 19 | 19 | 20 | 19 | 18 |

Example 5

The solution prepared from 3.113 kg of cryoprecipitate of human plasma (18,297 units of factor VIII) was applied to an affinity column (13.7 cm x 22.0 cm, 3.24 1) of monoclonal anti von-Willebrand antibody gel matrix, which was prepared by conjugation of 1.2 g of the antibody per liter of Sepharose® gel. The column was then washed with 3 column volumes of the factor VIII buffer of Example 4. Nineteen percent (3,537 units) of the factor VIII were not bound to the column. The column was eluted with 0.25 M calcium chloride in the factor VIII buffer. The factor VIII activity containing portion, 3.556 kg (9,880 units), was collected. The eluted factor VIII was applied on an Aminohexyl-Sepharose® column (2.5 cm x 5.6 cm, Pharmacia) immediately after a five-fold in-line dilution with the AH-Sepharose® equilibration buffer (20 mM histidine, 100 mM lysine hydrochloride, pH 6.8). The flow rate was 12 ml per minute.

There was no detectable factor VIII activity in the solution that passed through the AH-Sepharose® column. The column was washed with 209 g of 50 mM calcium chloride in the AH-Sepharose® equilibration buffer. A small amount of factor VIII activity (165 units, 1.7%) was detected in the wash buffer solution collected. The factor VIII was then eluted from the column with 500 mM calcium chloride in the AH-Sepharose® equilibration buffer. The peak fraction of the elution profile contained 6,890 units of factor VIII in 26.5 g. The eluted factor VIII was dialyzed overnight at 4°C against the formulation buffer solution composed of 1 M sodium chloride, 5mM calcium chloride, 3 mM histidine, 2% mannitol, pH 7.0. The dialyzed factor VIII had 266 u/ml (23 g).

Example 6

One kg frozen human plasma cryoprecipitate was placed in a solution of 2.8 kg of 0.055 M glycine and 0.038 M sodium chloride. The mixture was placed in a 37°C water bath and agitated under laminar flow of air to dissolve the cryoprecipitate. 0.1 N acetic acid was added dropwise to the suspension to bring the pH to 6.90 ± 0.1. 100 g of Rehsorptar® was added to the mixture, and agitated for 15 to 20 minutes at 35 to 37°C. The suspension was centrifuged at 4,000 x g at room temperature for 15 minutes and the supernatant was collected. The Rehsorptar® treatment was repeated. The supernatant (3.8 kg) was mixed with 0.94 kg of 5 M sodium chloride solution to make the mixture 1 M in sodium chloride. The mixture had 7.6 units factor VIII activity per ml (35,811 units total). 4.6 kg of the mixture (35,250 units) were loaded on a 7.1 1 (25 x 14.5 cm) size monoclonal anti von-Willebrand antibody column (1.2 g of the antibody conjugated per 1 of sepharose) at room temperature with a flow rate of 14 ml per minute. The column was washed with 11.6 kg of 1 M sodium chloride in the factor VIII buffer solution of Example 4. The non-bound factor VIII activity was 5,950 units. The absorbed factor VIII was eluted with 0.25 M calcium chloride in the factor VIII buffer. A total of 20,090 units of factor VIII was collected in 10.1 kg of eluate. The eluted factor VIII was chromatographed through an Aminohexyl-Sepharose® column (5 x 3 cm) which was previously equilibrated with 0.02 M histidine, 0.10 M lysine hydrochloride, pH 6.8. The factor VIII was applied on the AH-Sepharose® column at 4°C after 5-fold dilution with the AH-Sepharose® equilibration buffer. The flow rate was 44 ml per minute. All of the factor VIII activity was bound to the column. The column was washed with 420 g of 0.05 M calcium chloride in the above referred to equilibration buffer. 4,349 units of factor VIII were washed off. The factor VIII was then eluted from the column with 0.50 M calcium chloride in the equilibration buffer. The factor VIII activity (12,538 units) was collected in 90.81 g of the eluate. The isolated factor VIII was dialyzed overnight at 4°C against 1 M sodium chloride, 3 mM histidine, 5 mM calcium chloride, pH 7.0, with 2 changes. 12,621 units of factor VIII was recovered. The material was stored at 4°C for several days. To a sample of factor VIII solution, powdered mannitol was added to make 2% w/v in mannitol (83 units/ml). The mixture was lyophilized and reconstituted and 72 units per ml were recovered. To another sample of factor VIII solution, powdered maltose was added to make 2% w/v in maltose (96 units/ml). The mixture was lyophilized and reconstituted and 94 units per ml were recovered.

## Claims
### Claims for the following Contracting States : AT, BE, CH, DE, FR, GB, IT, LI, LU, NL, SE

1. A stable plasma protein formulation comprising in an aqueous solution:
   a plasma protein in a therapeutically effective amount;
   from 0.35 M to 1.2 M sodium chloride, potassium chloride, or mixtures thereof;
   from 1.5 mM to 40 mM calcium chloride; and
   from 1 mM to 50 mM histidine;
   said aqueous solution having a pH of from 6.0 to 7.6.

2. The formulation of claim 1 wherein said plasma protein is factor VIII.

3. The formulation of claim 1 or 2 wherein the plasma protein is present in unit dosage form.

4. The formulation of claim 3 wherein the factor VIII has a concentration from 2 to 500 units per ml.

5. The formulation of any of claims 1 to 4 wherein the formulation contains 1 M sodium chloride, potassium chloride or mixtures thereof;
   from 3.5 mM to 15 mM calcium chloride;
   from 2 mM to 10 mM histidine;
   said aqueous solution having a pH of from 6.0 to 7.6.

6. The formulation of any of claims 1 to 5 further comprising up to 10% w/v of a sugar selected from mannitol, sucrose or maltose.

7. A stable plasma formulation wherein the formulation is in lyophilized form and upon reconstitution with pyrogen-free water forms the aqueous formulation of claims 1 to 6.

8. A stable plasma protein in lyophilized form for use in preparing with pyrogen-free water the formulation of any of claims 1 to 6.

9. A composition for stabilizing from 2 ml to 2000 ml of an aqueous solution of a lyophilized plasma protein in a therapeutically effective amount, comprising:
   an amount of sodium chloride of potassium chloride or mixtures thereof to provide from 0.35 M to 1.2 M sodium chloride or potassium chloride or mixtures thereof when reconstitued in said aqueous solution;
   an amount of calcium chloride to provide from 1.5 mM to 40 mM calcium chloride when reconstituted in said aqueous solution;
   an amount of histidine to provide from 1 mM to 50 mM histidine when reconstituted in said aqueous solution.

**10.** A composition according to claim 9 wherein the plasma protein is in unit dosage form.

**11.** A composition according to claim 9 or 10 wherein the plasma protein is Factor VIII.

**Claims for the following Contracting States : ES, GR**

**1.** A method for the preparation of a stable plasma protein formulation, characterized by preparing an aqueous solution which comprises:
a plasma protein in a therapeutically effective amount;
from 0.35 M to 1.2 M sodium chloride, potassium chloride, or mixtures thereof;
from 1.5 mM to 40 mM calcium chloride; and
from 1 mM to 50 mM histidine;
said aqueous solution having a pH of from 6.0 to 7.6.

**2.** The method of claim 1 wherein said plasma protein is factor VIII.

**3.** The method of claim 1 or 2 wherein the plasma protein is present in unit dosage form.

**4.** The method of claim 3 wherein the factor VIII has a concentration from 2 to 500 units per ml.

**5.** The method of any of claims 1 to 4 wherein the formulation contains 1 M sodium chloride, potassium chloride or mixtures thereof;
from 3.5 mM to 15 mM calcium chloride;
from 2 mM to 10 mM histidine;
said aqueous solution having a pH of from 6.0 to 7.6.

**6.** The method of any of claims 1 to 5, further comprising up to 10% w/v of a sugar selected from mannitol, sucrose or maltose.

**7.** A method for the preparation of a stable plasma formulation, characterized in that the formulation is in lyophilized form and contains further additives so that upon reconstitution with pyrogen-free water the same forms the aqueous formulation of claims 1 to 6.

**8.** A method for the preparation of a stable plasma protein, characterized in that the formulation is in lyophilized form and contains further additives so that upon reconstitution with pyrogen-free water the same forms the aqueous formulation of claims 1 to 6.

**9.** A method for the preparation of a composition for stabilizing from 2 ml to 2000 ml of an aqueous solution of a lyophilized plasma protein in a therapeutically effective amount, characterized by the addition of:
an amount of sodium chloride or potassium chloride or mixtures thereof to provide from 0.35 M to 1.2 M sodium chloride or potassium chloride or mixtures thereof when reconstituted in said aqueous solution;
an amount of calcium chloride to provide from 1.5 mM to 40 mM calcium chloride when reconstituted in said aqueous solution;
an amount of histidine to provide from 1 mM to 50 mM histidine when reconstitued in said aqueous solution.

**10.** The method according to claim 9 wherein the plasma protein is in unit dosage form.

**11.** The method according to claim 9 or 10 wherein the plasma protein is factor VIII.

**Patentansprüche**
**Patentansprüche für folgende Vertragsstaaten : AT, BE, CH, DE, FR, GB, IT, LI, LU, NL, SE**

**1.** Stabiler Plasmaproteinansatz, umfassend in einer wässrigen Lösung:
ein Plasmaprotein in einer therapeutisch effektiven Menge;
von 0,35 bis 1,2 M Natriumchlorid, Kaliumchlorid oder Mischungen davon;

von 1,5 bis 40 mM Kalziumchlorid; und
von 1 bis 50 mM Histidin;
wobei die wässrige Lösung einen pH von 6,0 bis 7,6 aufweist.

2. Ansatz nach Anspruch 1, dadurch **gekennzeichnet,** dass das Plasmaprotein Faktor VIII ist.

3. Ansatz nach Anspruch 1 oder 2, dadurch **gekennzeichnet,** dass das Plasmaprotein in einer Einheitsdosierungsform vorhanden ist.

4. Ansatz nach Anspruch 3, dadurch **gekennzeichnet,** dass der Faktor VIII eine Konzentration von 2 bis 500 Einheiten/ml aufweist.

5. Ansatz nach einem der Ansprüche 1 bis 4, dadurch **gekennzeichnet,** dass der Ansatz 1 M Natriumchlorid, Kaliumchlorid oder Mischungen davon; von 3,5 bis 15 mM Kalziumchlorid, von 2 bis 10 mM Histidin enthält, wobei die wässrige Lösung einen pH von 6,0 bis 7,6 aufweist.

6. Ansatz nach einem der Ansprüche 1 bis 5, dadurch **gekennzeichnet,** dass er weiterhin bis zu 10 % G/V eines Zuckers umfasst, ausgewählt aus Mannit, Sucrose oder Maltose.

7. Stabiler Plasmaansatz, dadurch **gekennzeichnet,** dass der Ansatz in lyophilisierter Form vorliegt und aufgrund der Rückbildung mit pyrogenfreiem Wasser den wässrigen Ansatz nach den Ansprüchen 1 bis 6 bildet.

8. Stabiles Plasmaprotein in lyophilisierter Form zur Verwendung für die Herstellung des Ansatzes nach einem der Ansprüche 1 bis 6 mit pyrogenfreiem Wasser.

9. Zusammensetzung zum Stabilisieren von 2 bis 2000 ml einer wässrigen Lösung eines lyophilisierten Plasmaproteins in einer therapeutisch effektiven Menge, umfassend:
eine Menge an Natriumchlorid oder Kaliumchlorid oder Mischungen davon, zum Schaffen von 0,35 bis 1,2 M Natriumchlorid oder Kaliumchlorid oder Mischungen davon, wenn es in der wässrigen Lösung rückgebildet ist;
eine Menge an Kalziumchlorid zum Schaffen von 1,5 bis 40 mM Kalziumchlorid, wenn es in der wässrigen Lösung rückgebildet ist;
eine Menge an Histidin zum Schaffen von 1 bis 50 mM Histidin, wenn es in der wässrigen Lösung rückgebildet ist.

10. Zusammensetzung nach Anspruch 9, dadurch **gekennzeichnet,** dass das Plasmaprotein in Einheitsdosierungsform vorliegt.

11. Zusammensetzung nach Anspruch 9 oder 10, dadurch **gekennzeichnet,** dass das Plasmaprotein Faktor VIII ist.

**Patentansprüche für folgende Vertragsstaaten : ES, GR**

1. Verfahren zur Herstellung eines stabilen Plasmaproteinansatzes, **gekennzeichnet** durch Herstellen einer wässrigen Lösung, umfassend:
ein Plasmaprotein in einer therapeutisch effektiven Menge;
von 0,35 bis 1,2 M Natriumchlorid, Kaliumchlorid oder Mischungen davon;
von 1,5 bis 40 mM Kalziumchlorid; und
von 1 bis 50 mM Histidin;
wobei die wässrige Lösung einen pH von 6,0 bis 7,6 aufweist.

2. Verfahren nach Anspruch 1, dadurch **gekennzeichnet,** dass das Plasmaprotein Faktor VIII ist.

3. Verfahren nach Anspruch 1 oder 2, dadurch **gekennzeichnet,** dass das Plasmaprotein in einer Einheitsdosierungsform vorhanden ist.

4. Verfahren nach Anspruch 3, dadurch **gekennzeichnet,** dass der Faktor VIII eine Konzentration von 2

EP 0 314 095 B1

bis 500 Einheiten/ml aufweist.

**5.** Verfahren nach einem der Ansprüche 1 bis 4, dadurch **gekennzeichnet,** dass der Ansatz 1 M Natriumchlorid, Kaliumchlorid oder Mischungen davon; von 3,5 bis 15 mM Kalziumchlorid, von 2 bis 10 mM Histidin enthält, wobei die wässrige Lösung einen pH von 6,0 bis 7,6 aufweist.

**6.** Verfahren nach einem der Ansprüche 1 bis 5, dadurch **gekennzeichnet,** dass die Lösung weiterhin bis zu 10 % G/V eines Zuckers enthält, ausgewählt aus Mannit, Sucrose oder Maltose.

**7.** Verfahren zur Herstellung eines stabilen Plasmaansatzes, dadurch **gekennzeichnet,** dass der Ansatz in lyophilisierter Form vorliegt und weiterhin Additive enthält, so das er aufgrund der Rückbildung mit pyrogenfreiem Wasser den wässrigen Ansatz nach den Ansprüchen 1 bis 6 bildet.

**8.** Verfahren zur Herstellung eines stabilen Plasmaproteins, dadurch **gekennzeichnet,** dass der Ansatz in lyophilisierter Form vorliegt, und weiter Additive enthält, so dass er aufgrund der Rückbildung mit pyrogenenfreiem Wasser den wässrigen Ansatz nach einem der Ansprüche 1 bis 6 bildet.

**9.** Verfahren zur Herstellung einer Zusammensetzung zum Stabilisieren von 2 bis 2000 ml einer wässrigen Lösung eines lyophilisierten Plasmaproteins in einer therapeutisch effektiven Menge, **gekennzeichnet** durch die Zugabe von:
einer Menge an Natriumchlorid oder Kaliumchlorid oder Mischungen davon, zum Schaffen von 0,35 bis 1,2 M Natriumchlorid oder Kaliumchlorid oder Mischungen davon, wenn es in der wässrigen Lösung rückgebildet wird;
einer Menge an Kalziumchlorid zum Schaffen von 1,5 bis 40 mM Kalziumchlorid, wenn es in der wässrigen Lösung rückgebildet wird;
einer Menge an Histidin zum Schaffen von 1 bis 50 mM Histidin, wenn es in der wässrigen Lösung rückgebildet wird.

**10.** Verfahren nach Anspruch 9, dadurch **gekennzeichnet,** dass das Plasmaprotein in Einheitsdosierungsform vorliegt.

**11.** Verfahren nach Anspruch 9 oder 10, dadurch **gekennzeichnet,** dass das Plasmaprotein Faktor VIII ist.

**Revendications**

**Revendications pour les Etats contractants suivants : AT, BE, CH, DE, FR, GB, IT, LI, LU, NL, SE**

**1.** Formulation stable de protéine plasmatique, comprenant, en solution aqueuse :
  - une protéine plasmatique présente dans une quantité thérapeutiquement efficace ;
  - du chlorure de sodium, du chlorure de potassium, ou des mélanges de ceux-ci, de 0,35 M à 1,2 M ;
  - du chlorure de calcium de 1,5 mM à 40 mM ; et
  - de l'histidine de 1 mM à 50 mM ;
ladite solution aqueuse ayant un pH de 6,0 à 7,6.

**2.** Formulation selon la revendication 1, dans laquelle ladite protéine plasmatique est le facteur VIII.

**3.** Formulation selon l'une des revendications 1 et 2, dans laquelle la protéine plasmatique est présente sous une forme de dosage unitaire.

**4.** Formulation selon la revendication 3, dans laquelle le facteur VIII a une concentration de 2 à 500 unités par ml.

**5.** Formulation selon l'une quelconque des revendications 1 à 4, dans laquelle la formulation contient :
  - du chlorure de sodium, du chlorure de potassium, ou des mélanges de ceux-ci, 1 M ;
  - du chlorure de calcium de 3,5 mM à 15 mM ;
  - de l'histidine de 2 mM à 10 mM ;
ladite solution aqueuse ayant un pH de 6,0 a 7,6.

**6.** Formulation selon l'une quelconque des revendications 1 à 5, comprenant en outre jusqu'à 10% p/v d'un sucre choisi parmi le mannitol, le sucrose ou le maltose.

**7.** Formulation plasmatique stable, dans laquelle la formulation se présente sous une forme lyophilisée et, après reconstitution avec de l'eau apyrogène, forme la formulation aqueuse telle que définie à l'une des revendications 1 à 6.

**8.** Protéine plasmatique stable sous forme lyophilisée, destinée à être utilisée dans la préparation, avec de l'eau apyrogène, de la formulation telle que définie à l'une quelconque des revendications 1 à 6.

**9.** Composition pour la stabilisation de 2 ml à 2000 ml d'une solution aqueuse d'une protéine plasmatique lyophilisée, dans une quantité thérapeutiquement efficace, comprenant :
- une quantité de chlorure de sodium, ou de chlorure de potassium, ou de mélanges de ceux-ci, capable de fournir du chlorure de sodium, ou du chlorure de potassium, ou des mélanges de ceux-ci, de 0,35 M à 1,2 M, une fois reconstituée dans ladite solution aqueuse ;
- une quantité de chlorure de calcium capable de fournir du chlorure de calcium de 1,5 mM à 40 mM une fois reconstituée dans ladite solution aqueuse ;
- une quantité d'histidine capable de fournir de l'histidine de 1 mM à 50 mM une fois reconstituée dans ladite solution aqueuse.

**10.** Composition selon la revendication 9, dans laquelle la protéine plasmatique se présente sous une forme de dosage unitaire.

**11.** Composition selon l'une des revendications 9 et 10, dans laquelle la protéine plasmatique est le facteur VIII.

**Revendications pour les Etats contractants suivants : ES, GR**

**1.** Procédé de préparation d'une formulation stable de protéine plasmatique, caractérisé par la préparation d'une solution aqueuse qui comprend :
- une protéine plasmatique présente dans une quantité thérapeutiquement efficace ;
- du chlorure de sodium, du chlorure de potassium, ou des mélanges de ceux-ci de 0,35 M à 1,2 M ;
- du chlorure de calcium de 1,5 mM à 40 mM : et
- de l'histidine de 1 mM à 50 mM ;
ladite solution aqueuse ayant un pH de 6,0 à 7,6.

**2.** Procédé selon la revendication 1, dans lequel ladite protéine plasmatique est le facteur VIII.

**3.** Procédé selon l'une des revendications 1 et 2, dans lequel la protéine plasmatique est présente sous une forme de dosage unitaire.

**4.** Procédé selon la revendication 3, dans lequel le facteur VIII a une concentration de 2 à 500 unités par ml.

**5.** Procédé selon l'une quelconque des revendications 1 à 4, dans lequel la formulation contient :
- du chlorure de sodium, du chlorure de potassium, ou des mélanges de ceux-ci, 1 M ;
- du chlorure de calcium de 3,5 mM à 15 mM ;
- de l'histidine de 2 mM à 10 mM ;
ladite solution aqueuse ayant un pH de 6,0 à 7,6.

**6.** Procédé selon l'une quelconque des revendications 1 à 5, comprenant en outre jusqu'à 10% p/v d'un sucre choisi parmi le mannitol, le sucrose ou le maltose.

**7.** Procédé de préparation d'une formulation plasmatique stable, caractérisé par le fait que la formulation se présente sous une forme lyophilisée et contient d'autres additifs de telle sorte qu'après reconstitution avec de l'eau apyrogène, celle-ci forme la formulation aqueuse telle que définie à l'une des revendications 1 à 6.

**8.** Procédé de préparation d'une protéine plasmatique stable, caractérisé par le fait que la formulation se présente sous une forme lyophilisée, et contient d'autres additifs de telle sorte qu'après reconstitution avec de l'eau apyrogène, celle-ci forme la formulation aqueuse telle que définie à l'une des revendications 1 à 6.

**9.** Procédé de préparation d'une composition pour la stabilisation de 2 ml à 2000 ml d'une solution aqueuse d'une protéine plasmatique lyophilisée, dans une quantité thérapeutiquement efficace, caractérisé par l'addition :
- d'une quantité de chlorure de sodium, ou de chlorure de potassium, ou de mélanges de ceux-ci, capable de fournir du chlorure de sodium, ou du chlorure de potassium, ou des mélanges de ceux-ci, de 0,35 M à 1,2 M, une fois reconstituée dans ladite solution aqueuse ;
- d'une quantité de chlorure de calcium capable de fournir du chlorure de calcium de 1,5 mM à 40 mM une fois reconstituée dans ladite solution aqueuse ;
- d'une quantité d'histidine capable de fournir de l'histidine de 1 mM à 50 mM une fois reconstituée dans ladite solution aqueuse.

**10.** Procédé selon la revendication 9, dans lequel la protéine plasmatique se présente sous une forme de dosage unitaire.

**11.** Procédé selon l'une quelconque des revendications 9 et 10, dans lequel la protéine plasmatique est le facteur VIII.